Europäisches Patentamt

European Patent Office

Office européen des brevets

(11) Numéro de publication: **0 380 422**
**A1**

# DEMANDE DE BREVET EUROPEEN

(21) Numéro de dépôt: **90400220.1**

(51) Int. Cl.5: **A61K 37/02, C07K 3/28**

(22) Date de dépôt: **25.01.90**

(30) Priorité: **27.01.89 FR 8901026**

(43) Date de publication de la demande:
**01.08.90 Bulletin 90/31**

(84) Etats contractants désignés:
**AT BE CH DE DK ES FR GB GR IT LI LU NL SE**

(71) Demandeur: **FONDATION NATIONALE DE TRANSFUSION SANGUINE**
**6, rue Alexandre Cabanel**
**F-75739 Paris Cédex 15(FR)**

(72) Inventeur: **Boffa, Georges**
**62, chemin de Vauhallan**
**F-91120 Palaiseau(FR)**

(74) Mandataire: **Warcoin, Jacques et al**
**Cabinet Régimbeau 26, avenue Kléber**
**F-75116 Paris(FR)**

(54) **Procédé de préparation de protéines inhibitrices de la lymphocytotoxicité et du complément et protéines obtenues.**

(57) La présente invention concerne un procédé de préparation d'une protéine inhibitrice de la lymphocytotoxicité et du complément, caractérisé en ce qu'on isole ladite protéine de la fraction I, III ou de la fraction I, II, III de Cohn.

EP 0 380 422 A1

# PROCEDE DE PREPARATION DE PROTEINES INHIBITRICES DE LA LYMPHOCYTOTOXICITE ET DU COMPLEMENT ET PROTEINES OBTENUES

La présente invention concerne un procédé de préparation d'une protéine de mammifère ayant une activité du type de celle présentée par la protéine inhibitrice du rejet de greffe (protéine IRG) décrite dans le brevet WO 87/02368.

Le brevet WO 87/02368 décrit une nouvelle protéine caractérisée, notamment, par le fait qu'elle inhibe de façon significative, in vitro, l'activité des lymphocytes cytotoxiques au cours d'une réaction lymphocytaire mixte secondaire et que, mélangée avec le complément, elle inhibe la réaction d'hémolyse des globules rouges de moutons sensibilisés. Cette nouvelle protéine, administrée in vivo, prolonge de façon significative la survie des allogreffes.

L'intérêt de ce type de protéine est considérable, notamment pour la prévention du rejet des greffes.

La protéine IRG décrite dans le brevet précédent est obtenue par fractionnement de sérums de mammifères femelles gestantes et/ou d'autres animaux mammifères développant une réaction inflammatoire expérimentale.

Dans ce procédé, à partir de ces sérums, on isole les fractions migrant avec les alpha-2-macroglobulines et on procède à un épuisement immunologique avec des anticorps IgG anti-sérum entier de mâle et/ou anti-(alpha-2-macroglobulines) de mâle pour recueillir les fractions ne réagissant pas avec lesdits anticorps.

Or, la présente invention repose sur la mise en évidence de protéines du type IRG, et plus particulièrement de protéines inhibitrices de la lymphocytotoxicité et du complément, qui peuvent être extraites de sérums ou de plasmas normaux et non pas uniquement de sérums ou de plasmas de mammifères femelles gestantes et/ou d'animaux mammifères développant des réactions inflammatoires expérimentales.

Plus particulièrement, la présente invention concerne un procédé de préparation d'une protéine inhibitrice de la lymphocytotoxicite et du complément, caractérisée en ce qu'elle est isolée de la fraction I, III ou de la fraction I, II, III de Cohn, plus particulièrement lorsque ces fractions de Cohn sont obtenues à partir de sérums ou de plasmas normaux.

Le fractionnement de Cohn est un procédé de fractionnement dans lequel le plasma est cryoprécipité, le surnageant étant prélevé, il est précipité à l'alcool à 19 % à un pH de 5,85, le précipité correspondant étant appelé fraction I + II + III.

La fraction I + II + III est de nouveau précipitée à l'alcool à 13 % à un pH de 4,8 puis à un pH de 5,1 pour donner un précipité qui correspond à la fraction I + III.

C'est normalement le surnageant de cette dernière précipitation qui est traité pour donner la fraction II, laquelle contient les gamma-globulines qui constituent l'un des éléments utilisés industriellement dans ce fractionnement du plasma.

Jusqu'à maintenant, la fraction I + III est en principe éliminée.

La fraction I + II + III est en général congelée à - 20° C pour être conservée.

C'est dans ces fractions I + II + III et I + III qu'ont été mises en évidence les protéines selon la présente invention.

L'activité de ces fractions en elle-même est déjà importante mais il est bien entendu possible de les fractionner pour en extraire une fraction contenant les alpha-2-macroglobulines qui contiennent la protéine inhibitrice de la lymphocytotoxicité et du complément.

Ce type de fractionnement peut être effectué par différentes techniques, notamment de type chromatographique, en particulier en utilisant un gel filtrant AcA 22 comme cela sera montré plus précisément dans les exemples, ou bien d'autres résines telles que la résine Sépharose CL 6B.

D'une façon générale, les protéines selon la présente invention peuvent être obtenues par :
- fractionnement de sérums de mammifères non gestants de façon à isoler la fraction migrant avec les alpha-2-macroglobulines, et
- separation par chromatographie de la fraction de l'alpha-2-macroglobuline présentant l'activité inhibitrice de la lymphocytotoxicité et du complément.

Dans le premier type de fractionnement qui a été évoqué, à savoir le fractionnement de Cohn, les précipitations sont effectuées à l'alcool. On peut, à partir des sérums, précipiter les alpha-2-macroglobulines à l'aide de rivanol, les alpha-2-macroglobulines se retrouvant dans le précipité, ledit précipité pouvant être repris pour être séparé par chromatographie comme cela a été indiqué précédemment.

Les protéines selon la présente invention présentent des propriétés d'inhibition de la lymphocytotoxicité et une activité inhibitrice du complément qui font qu'elles sont très semblables à la protéine IRG et à ses analogues qui ont été décrits dans le brevet WO 87/02368.

Les applications thérapeutiques sont donc du même type que celles qui ont été mentionnées dans le brevet précédent.

Les exemples ci-après permettront de mieux mettre en évidence d'autres caractéristiques et avantages de la présente invention.

Exemple 1

Les alpha-2-macroglobulines sériques sont purifiées par précipitation en présence de rivanol suivie d'une chromatographie d'exclusion sur gel de sépharose. Les alpha-2-macroglobulines ont été comparées chez l'homme et la femme gestante.

Un mélange de sérum humain de 60 ml a été additionné de "soybean trypsin inhibitor" (STI) et de phényl méthane sulfone fluorure (PMSF) puis dilué en tampon $K_2HPO_4$, $KH_2PO_4$ 30 mM, pH 7,2.

Une poudre de rivanol est ajoutée sous agitation douce jusqu'à une concentration finale de 0,4 % à la température ambiante. Après centrifugation, le culot est repris en NaCl 0,83 M pour précipiter le rivanol. Un passage sur colonne de G 25 coarse élimine les contaminants résiduels de rivanol. Une colonne de sépharose CL 6B équilibrée en tampon phosphate Na/K, pH 7,2 est utilisée avec un rapport ml de gel/mg de protéine de 3,4.

Cinq fractions sont obtenues, les alpha-2-macroglobulines se distribuent dans les fractions 2 et 3.

Le tableau 1 montre que l'activité inhibitrice du complément se distribue dans les macromolécules issues soit du sérum d'homme normal, soit du sérum de femmes gestantes, avec un maximum dans la fraction 3.

La fraction inhibitrice de lyse cellulaire lymphocyte dépendant se situe essentiellement dans la fraction 2 issue de sérum d'homme normal (SHN) ou de sérum de femme gestante.

Tableau 1

| Répartition de l'activité inhibitrice du complément et de l'activité anti-cytotoxique dans les fractions de Sépharose CL 6B | | | | | |
|---|---|---|---|---|---|
| | 1 | 2 | 3 | 4 | 5 |
| S.H.N. | | | | | |
| Activité inhibitrice complément/Unités | 79 230 | 29 120 | 177 920 | 0 | 0 |
| Activité anticytotoxique 0,3 A/ml (%) | - | 95% | 3% | 0 | - |
| Sérum femme gestante | | | | | |
| Activité inhibitrice complément/Unités | 34 200 | 5 890 | 54 600 | 0 | 0 |
| Activité anticytotoxique 0,9 A/ml (%) | - | 100% | 2% | - | - |

Exemple 2

Les alpha-2-macroglobulines sont décelées en abondance dans des fractions obtenues par précipitation alcoolique selon la filière de Cohn.

Les activités immunomodulatrices sont recherchées à 5 niveaux :
. le plasma,
. le sérum issu de ce plasma par recalcification et addition de thromboplastine,
. la fraction I, II, III,
. la fraction IV,
. la fraction I, III.

Chaque échantillon est analysé avant dialyse, puis après dialyse et dans ce cas avec ou sans inhibiteurs.

Une activité inhibitrice du complément est décelée le long de la filière de fractionnement au niveau de la fraction I, II, III et en aval dans la fraction I, III (tableau 2).

Une inhibition de la lymphocytotoxicité (tableau 3) est décelée dans la fraction I, III. La fraction 1V est dépourvue de toute activite.

Le sérum issu par recalcification du plasma, les fractions I, II, III et I, III sont traitées par filtration sur AcA 22 en tampon phosphate de sodium 50 mM, NaCl 250 mM, pH 7,8 à 4°C sur une colonne 16 mm de diamètre contenant 220 ml de gel avec un débit de 5 ml/heure.

Des fractions de 7 ml sont recueillies et l'activité inhibitrice du complément y est dosée (AIC).

Le sérum révèle une seule zone d'élution du facteur actif au niveau des alpha-2-macroglobulines.

Les fractions I, II, III donnent deux zones dont l'une se superpose à la fraction alpha-2-macroglobuline, l'autre de plus faible taille moléculaire correspondant à la sous-unité de l'alpha-2-macroglobuline. Les fractions I et III donnent une seule zone d'élution au niveau de l'alpha-2-macroglobuline avec un rendement d'environ 12 % par apport à la fraction I, III de départ. Une inhibition de la lyse cellulaire lymphocyte dépendante est obtenue au niveau de la fraction macroglobulinique de I, II, III et à un degré plus faible au niveau des macroglobulines de la fraction industrielle I, III (tableau 4).

Les essais du tableau 4 ont été réalisés à partir de deux échantillons différents (3) et (5) fractionnés sur AcA 22.

## Activité anticytotoxique

Une méthode issue de celle de Solliday et Bach a été adaptée et mise au point avec des cellules mononuclées de donneurs de sang à groupe HLA non déterminé.

La stimulation des lymphocytes T se fait en coculture des deux populations cellulaires A et B issues de donneurs différents. Les cellules B ont subi une irradiation gamma préalable pour déclencher une réaction lymphocytaire T unidirectionnelle. En 7 jours, on obtient une population cellulaire enrichie en cellules T stimulées ABc.

La cytotoxicité des cellules ABc s'exprime vis-à-vis des cellules B cultivées parallèlement et stimulées par la PHA, puis marquées au [51]chrome.

L'inhibition des effets cytotoxiques est évaluée comme suit dans les tableaux 3 et 4 :

- B correspond au relargage spontané de [51]chrome,
- MAX, témoin de cytotoxicité maximum, correspond à un relargage total de [51]chrome, les cellules étant traitées par HCl,
- CsA est un témoin positif mettant en oeuvre la cyclosporine, inhibiteur de la cytotoxicité des lymphocytes T stimulés connus,
- comparaison avec différentes fractions.

Le pourcentage de cytotoxicité doit être de préférence supérieur à 30 %.

Tableau 2

Activité Inhibitrice du Complément (AIC)

| | Volume de départ (ml) | | Volume traité (ml) | AIC (U/ml) | AIC totale (U) | DO (UA/ml) | DO totale (U) | AIC totale (U/ml plasma) |
|---|---|---|---|---|---|---|---|---|
| Echantillon n° 1 (plasma) | 46 | sans inhibiteurs | 26 | 0 | 0 | 56,2 | 1 461,20 | |
| | | avec inhibiteurs | 20 | 0 | 0 | | | |
| Echantillon n° 2 (sérum issu de plasma+CaCl$_2$ | 26 | sans inhibiteurs | 11 | 0 | 0 | 54,5 | 599,5 | |
| | | avec inhibiteurs | 15 | 800 | 12 000 | | | |
| Echantillon n° 3 (I+II+III) | 10,5 | avant dialyse | 5 | 18 000 | 90 000 | | | 16 822 |
| | | sans inhibiteurs | 2,5 | 8 100 | 20 250 | 23,7 | 59,25 | 7 570 |
| | | avec inhibiteurs | 1 | 7 900 | 7 900 | | | 7 383 |

EP 0 380 422 A1

## Tableau 2    (SUITE)

### Activité Inhibitrice du Complément (AIC)

| | Volume de départ (ml) | | Volume traité (ml) | AIC (U/ml) | AIC totale (U) | DO (UA/ml) | DO totale (U) | AIC totale (U/ml plasma) |
|---|---|---|---|---|---|---|---|---|
| Echantillon | | avant dialyse | 10 | 0 | 0 | | | |
| n° 4 | 25,98 | sans inhibiteurs | 4 | 0 | 0 | 9,6 | 38,4 | |
| (IV) | | avec inhibiteurs | 6 | 0 | 0 | | | |
| Echantillon | | avant dialyse | 10 | 7 200 | 72 000 | | | 1 166 |
| n° 5 | 21,13 | sans inhibiteurs | 2,5 | 6 800 | 17 000 | 11,6 | 29 | 1 102 |
| (I+III) | | avec inhibiteurs | 3,5 | 5 200 | 18 200 | | | 842 |

Tableau 3

| Effet des fractions plasmatiques industrielles (sans inhibiteurs) sur la réaction de lymphocytotoxicité | | | | | | | | |
|---|---|---|---|---|---|---|---|---|
| Cellules 1.10⁶/ml | B | MAX | CsA | ABc/B | | | | |
| Echantillon testé | - | - | CsA 0,1µg/ml | AI 0,3UA/ml | BI 0,3UA/ml | CI 0,3UA/ml | DI 0,3UA/ml | EI 0,3UA/ml |
| Rendement J7/J0 (%) | 162 | 171 | 81 | 193 | 166 | 157 | 202 | 105 |
| N cpm | 1 126 | 3 427 | 1 609 | 3 200 | 3 261 | 2 812 | 3 228 | 2 399 |
| Cytotoxicité (%) | - | 75 | 16 | 68 | 70 | 55 | 69 | 42 |
| Inhibition (%) | - | - | 79 | 11 | 8 | 14 | 9 | 45 |

cpm totaux moyens = 5 766 cpm = rendement marquage = 12 %

Relargage spontané = 20 %

relargage maximum = 4 168 cpm

AI : plasma

BI : sérum

CI : précipités I + II + III

DI : précipité IV

EI : précipités I + III

Tableau 4

Effet des fractions AcA 22 des échantillons 3 et 5 sur la lymphocytotoxicité

| Cellules | B | ABc/B | | ABc/B | | | |
|---|---|---|---|---|---|---|---|
| Echantillon testé | - | témoin | CsA | PI AcA22 éch. 3 | PII AcA22 éch. 3 | PI AcA22 éch. 5 | PII AcA22 éch. 5 |
| Doses | - | - | 0,1µg/ml | 0,3UA/ml | 0,3UA/ml | 0,3UA/ml | 0,3UA/ml |
| Rdt 37/30 (%) | 126 | 108 | 49 | 124 | 79 | 133 | 122 |
| Rdt (%) $\frac{\text{ABc fraction J7}}{\text{ABc témoin J7}}$ | - | 100 | 45 | 115 | 73 | 123 | 113 |
| N cpm | 2 555,5 | 8 586 | 3 118 | 5 322 | 7 338 | 6 635 | 7 674 |
| Cytotoxicité (%) | - | 66 | 6 | 30 | 52 | 44 | 56 |
| Inhibition (%) | - | - | 91 | 54 | 21 | 33 | 15 |

Cpm totaux moyens = 14 821 cpm
Rendement marquage = 24 %
Relargage maximum (HCl 6N) = 11 733,5 cpm
Relargage spontané = 22 %

8

**Revendications**

1) Procédé de préparation d'une protéine inhibitrice de la lymphocytotoxicité et du complément, caractérisé en ce qu'on isole ladite protéine de la fraction I, III ou de la fraction I, II, III de Cohn issue de plasmas humains normaux.

2) Procédé selon la revendication 1, caractérisé en ce que la fraction I, III ou I, II, III est fractionnée pour en extraire la fraction contenant les alpha-2-macroglobulines qui contiennent la protéine inhibitrice de la lymphocytotoxicité et du complément.

3) Procédé selon la revendication 2, caractérisé en ce que les fractions de Cohn sont fractionnées avec un gel filtrant AcA 22 pour séparer la fraction contenant les alpha-2-macroglobulines.

4) Procédé de préparation d'une protéine inhibitrice de la lymphocytotoxicité et du complément, caractérisé en ce que :
- on fractionne le sérum de mammifère non gestant de façon à isoler les fractions migrant avec les alpha-2-macroglobulines,
- on sépare par chromatographie la fraction d'alpha-2-macroglobuline présentant l'activité inhibitrice de la lymphocytotoxicité.

5) Procédé selon la revendication 4, caractérisé en ce que les fractions migrant avec les alpha-2-macroglobulines sont obtenues par précipitation du sérum en présence de rivanol, les fractions en cause étant dans le précipité.

6) Procédé selon l'une des revendications 4 et 5, caractérisé en ce que les fractions d'alpha-2-macroglobuline sont séparées par chromatographie sur une colonne de AcA 22 ou de Sépharose CL 6B.

7) Protéine pouvant être obtenue selon le procédé d'une des revendications 1 à 6 et présentant in vitro une activité inhibitrice de la lymphocytotoxicité et une activité inhibitrice du complément.

Office européen des brevets

# RAPPORT DE RECHERCHE EUROPEENNE

Numero de la demande

EP 90 40 0220

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | WO-A-8 702 368 (CENTRE NATIONAL DE LA RECHERCHE SCENTIFIQUE) * Page 2, lignes 12-36; page 4, lignes 1-18; page 10, lignes 1-21; page 21, lignes 29-33 * --- | 1-7 | A 61 K 37/02 C 07 K 3/28 |
| Y | FR-A-2 460 137 (INSTITUT MERIEUX) * Page 3, lignes 35-40; page 4, lignes 1-39; page 5, lignes 1-39; pages 6-7, exemples 1-2 * ----- | 1-7 | |

**DOMAINES TECHNIQUES RECHERCHES (Int. Cl.5)**

A 61 K

Le présent rapport a été établi pour toutes les revendications

| Lieu de la recherche | Date d'achèvement de la recherche | Examinateur |
|---|---|---|
| LA HAYE | 02-05-1990 | FERNANDEZ Y BRANAS F.J. |